# EUROPEAN PATENT APPLICATION

(11) **EP 3 997 982 A1**
(43) Date of publication of application: **18.05.2022**
(21) Application number: 20836620.3
(22) Date of filing: 15.06.2020
(51) Int. Cl.: A01N 43/50, A01N 25/02, A01P 21/00

(54) **PLANT GROWTH REGULATOR AND METHOD FOR PROMOTING PLANT GROWTH**

(30) Priority: 10.07.2019 JP 2019128636
(71) Applicant: Kureha Corporation, Chuo-ku Tokyo 103-8552 (JP)
(72) Inventor: KOSHIYAMA, Tatsuyuki, Tokyo 103-8552 (JP); EIZUKA, Takayoshi, Tokyo 103-8552 (JP)
(74) Representative: Hoefer & Partner Patentanwälte mbB
(86) International application number: PCT/JP2020/023333
(87) International publication number: WO 2021/005970

(57) **Abstract**

In order to provide a plant growth regulator with an excellent plant growth promoting effect, the plant growth regulator of the present invention includes a compound represented by Formula (I) or its tautomer, or an agrochemically acceptable salt thereof. 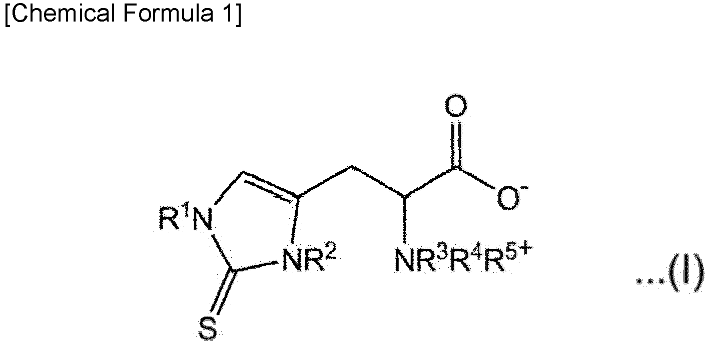 where R¹ and R² each independently represent a hydrogen atom or an alkyl group having from 1 to 4 carbon atoms, and R³ to R⁵ each independently represent an alkyl group having from 1 to 4 carbon atoms.

## Description

### [TECHNICAL FIELD]

The present invention relates to a plant growth regulator and use thereof.

### [BACKGROUND ART]

There has been a demand for an agent having a high plant growth promoting effect on a wide range of plants. Examples of such agents include oxidized glutathione (Patent Document 1).

### [CITATION LIST]

### [PATENT DOCUMENT]

Patent Document 1: WO 2008/072602

### [SUMMARY OF INVENTION]

### [TECHNICAL PROBLEM]

However, existing agents are not sufficient in their growth promoting effect, and there is still a demand for a plant growth regulator exhibiting an excellent growth promoting effect. Therefore, the present invention has been made in view of the above problem, and intended to provide a plant growth regulator excellent in the plant growth promoting effect.

### [SOLUTION TO PROBLEM]

As a result of diligent studies by the present inventors, surprisingly, it was found that an excellent plant growth promoting effect can be obtained by using a specific antioxidant, which has been conventionally used for foods, medical products, and cosmetics, as a plant growth regulator, and thus the present invention has been completed. That is, in order to solve the problems described above, the plant growth regulator according to the present invention includes a compound represented by Formula (I) or its tautomer, or an agrochemically acceptable salt thereof as an active ingredient: 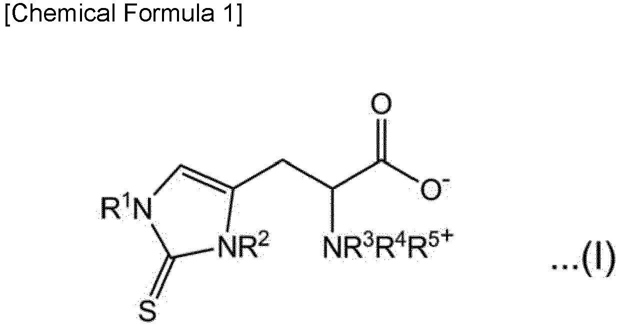 where in Formula (I), R¹ and R² each independently represent a hydrogen atom or an alkyl group having from 1 to 4 carbon atoms, and R³ to R⁵ each independently represent an alkyl group having from 1 to 4 carbon atoms.

The method for promoting plant growth according to the present invention includes treating a plant with the compound represented by Formula (I) or its tautomer, or an agrochemically acceptable salt thereof.

### [ADVANTAGEOUS EFFECTS OF INVENTION]

According to the present invention, a plant growth regulator having an excellent plant growth promoting effect can be provided.

### [DESCRIPTION OF EMBODIMENTS]

### [Plant growth regulator]

### (Active ingredients)

The plant growth regulator according to the present embodiment includes a compound represented by Formula (I) (hereinafter simply referred to as "compound (I)") or its tautomer, or an agrochemically acceptable salt thereof as an active ingredient: 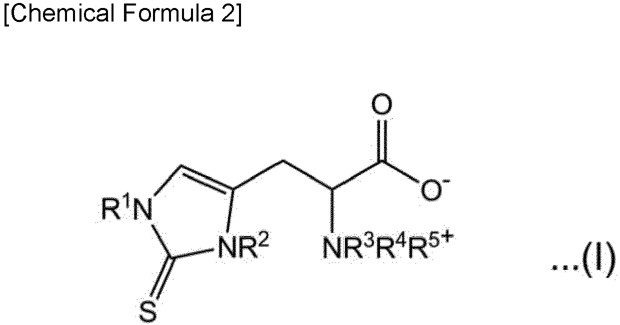 where in Formula (I), R¹ and R² each independently represent a hydrogen atom or an alkyl group having from 1 to 4 carbon atoms. R³ to R⁵ each independently represent an alkyl group having from 1 to 4 carbon atoms.

The alkyl group may be linear or branched, that is, may be a methyl group, an ethyl group, a propyl group, an isopropyl group, a butyl group, an isobutyl group, a sec-butyl group or a tert-butyl group.

At least one of R¹ and R² is preferably a hydrogen atom, and more preferably both are hydrogen atoms. When R¹ and R² are alkyl groups, they are preferably methyl groups, ethyl groups or propyl groups, more preferably methyl groups or ethyl groups, and even more preferably methyl groups.

R³ to R⁵ are each preferably independently a methyl group, an ethyl group or a propyl group, more preferably a methyl group or an ethyl group, and even more preferably a methyl group. At least one of R³ to R⁵ is preferably a methyl group, more preferably at least two are methyl groups, and even more preferably all are methyl groups.

"Its tautomer" refers to a tautomer of compound (I). Compound (I) has a tautomer when at least one of R¹ and R² is a hydrogen atom. More specifically, when R² is a hydrogen atom in Formula (I), the compound represented by Formula (II) below (hereinafter simply referred to as "compound (II)") can exist as a tautomer. In addition, when R¹ is a hydrogen atom in Formula (I), the compound represented by Formula (III) below (hereinafter simply referred to as "compound (III)") can exist as a tautomer. Compounds (II) and (III) are hereinafter collectively referred to simply as "tautomers". 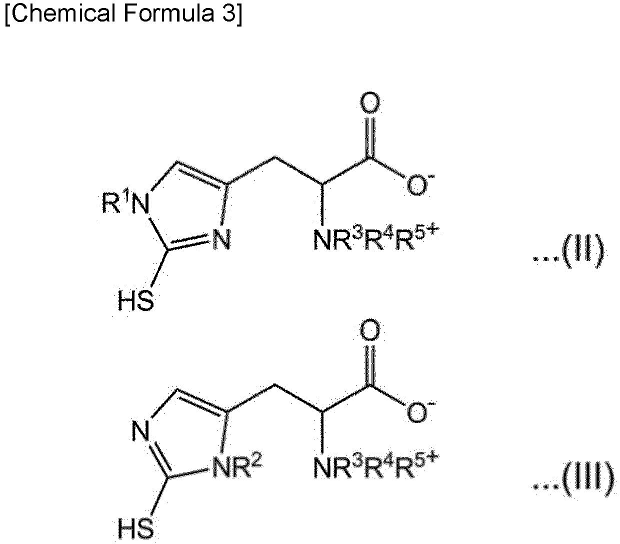 where in Formulas (II) and (III), R¹ to R⁵ are the same as R¹ to R⁵ in Formula (I).

The preferred compound as compound (I) or its tautomer is specifically ergothioneine, and L-(+)-ergothioneine is more preferred.

As these compounds, commercially available compounds may be used, or those synthesized by a technique well known to those skilled in the art, for example, a technique described in JP 2013-506706 T or JP 2006-160748 A may be used. Ergothioneine is known to be produced by bacteria and fungi. Examples of the production method using such a microorganism include the methods described in JP 2012-105618 A, JP 2014-223051 A, WO 2016/104437, WO 2016/121285, WO 2015/168112, and WO 2017/150304. As ergothioneine, a culture containing ergothioneine obtained from these microorganisms may be used as it is, or ergothioneine may be concentrated or purified before use.

"Agrochemically acceptable" usually means those are safe, non-toxic, and not undesired biologically or otherwise, but those acceptable for pesticides, especially for pesticides that promote plant growth.

The "agrochemically acceptable salt" of compound (I) or its tautomer is a salt that is agrochemically acceptable as defined above, and means those can provide the action and effect of compound (I) or its tautomer. Examples of such salts include hydrates, solvates, acid addition salts, salts formed when the acidic proton present in compound (I) or its tautomer are replaced with metal ions, and salts formed when the acidic proton coordinates with an organic base or an inorganic base.

The acid addition salt may be formed with an inorganic acid or an organic acid. Examples of the inorganic acids include hydrochloric acid, hydrobromic acid, sulfuric acid, nitric acid, and phosphoric acid. Examples of the organic acids include acetic acid, benzenesulfonic acid, benzoic acid, camphorsulfonic acid, citric acid, ethanesulfonic acid, fumaric acid, glucoheptonic acid, gluconic acid, glutamic acid, glycolic acid, hydroxynaphthoic acid, 2-hydroxyethanesulfonic acid, lactic acid, maleic acid, malic acid, mandelic acid, methanesulfonic acid, muconic acid, 2-naphthalenesulfonic acid, propionic acid, salicylic acid, succinic acid, dibenzoyl-L-tartaric acid, tartaric acid, p-toluenesulfonic acid, trimethylacetic acid, and trifluoroacetic acid.

Examples of the metal ion capable of substituting the acidic proton present in compound (I) or its tautomer include an alkali metal ion, an alkaline earth metal ion, and an aluminum ion.

Examples of the organic base capable of coordinating with the acidic proton present in compound (I) or its tautomer include diethanolamine, ethanolamine, N-methylglucamine, triethanolamine, and tromethamine. Examples of the inorganic base include aluminum hydroxide, calcium hydroxide, potassium hydroxide, sodium carbonate, and sodium hydroxide.

The plant growth regulator according to the present embodiment includes compound I or its tautomer, or an agrochemically acceptable salt thereof as an active ingredient, thereby exhibiting an excellent growth promoting effect in a plant treated with the regulator. In the present specification, "excellent in growth promoting effect" means that at least one of the plant growth indices is superior to known compounds. Examples of the "plant growth index" include plant height, number of tillers, number of flowers, number of fruits, and seed yield.

The plant growth regulator according to the present embodiment preferably includes a compound represented by Formula (I) or an agrochemically acceptable salt thereof as an active ingredient. The plant growth regulator according to the present embodiment may include a plurality of compounds among compound (I) or its tautomer, or an agrochemically acceptable salt thereof as an active ingredient.

Normally, in a solution, compound (I) and compound (II) or (III) can exist in equilibrium. The ratio of compound (I) to compound (II) or (III) can vary depending on the solvent, temperature, pH, or the like.

### (Applicable plant)

The plant growth regulator in the present embodiment generally exhibits a growth promoting effect on all plants, and examples of applicable plants include the following: Poaceae such as rice, wheat, barley, rye, oats, triticale, corn, sorghum, sugar cane, turf, bentgrass, bermudagrass, fescue, and ryegrass; legumes such as soybean, peanut, kidney bean, peas, adzuki beans, and alfalfa; Convolvulaceae such as sweet potatoes; solanaceae such as capsicum, pepper, tomato, eggplant, potato, and tobacco; Polygonaceae such as buckwheat; Asteraceae such as sunflower; Araliaceae such as ginseng; Brassicaceae such as rapeseed, Chinese cabbage, turnip, cabbage, and Japanese radish; Chenopodiaceae such as sugar beet; Malvaceae such as cotton; Rubiaceae such as coffee tree; Sterculiaceae such as cacao; Camellia such as tea; Cucurbitaceae such as watermelon, melon, cucumber, and pumpkin; Liliaceae such as onion, leeks, and garlic; Rosaceae such as strawberries, apples, almonds, apricots, Japanese apricots, cherry, plums, peaches, and pears; Apiaceae such as carrots; Araceae such as taro; Larvae such as mango; Pineapples such as pineapples; Carica such as papayas; Ebenaceae such as persimmons; Ericaceae such as blueberries, walnuts such as pecans; Musaceae such as bananas; Oleaceae such as olives; Palmae such as coconut, and date; Rutaceae such as mandarin orange, orange, grapefruit, and lemon; Vitaceae such as grapes; flowers and ornamental plants, trees other than fruit trees; and other ornamental plants.

Other examples include wild plants, cultivars, plants and cultivars bred by conventional hybridizing or plasmogamy, and genetically recombinant plants and cultivars obtained by gene manipulation. Examples of genetically recombined plants and cultivars include herbicide-tolerant crops, pest-resistant crops in which an insecticidal protein-producing gene has been recombined, pathogen-resistant crops in which a pathogen resistance derivative-producing gene has been recombined, taste-improved crops, yield-improved crops, preservation-improved crops, and yield-improved crops. Examples of genetically recombined cultivar that has been approved in each country include those stored in the database of the International Service for the Acquisition of Agri-biotech Applications (ISAAA). Specific examples include those containing trade names such as Roundup Ready, Liberty Link, IMI, SCS, Clearfield, Enlist, B.t., BXN, Poast Compatible, AgriSure, Genuity, Optimum, Powercore, DroughtGard, YieldGard, Herculex, WideStrike, Twinlink, VipCot, GlyTol, Newleaf, KnockOut, BiteGard, BtXtra, StarLink, Nucotn, NatureGard, Protecta, SmartStax, Power Core, InVigor, and Bollgard.

### (Formulation)

The plant growth regulator is generally prepared by mixing compound (I) or its tautomer, which is an active ingredient, or a mixture thereof, with a solid carrier or a liquid carrier (diluent), a surfactant, and other formulation aid and the like, and formulating the mixture into various forms such as a dustable powder, a wettable powder, a granule, and an emulsifiable concentrate for use.

Examples of the solid carrier, liquid carrier, and surfactant used as formulation aids are as follows. First, examples of the solid carrier include powder carriers and granular carriers such as minerals such as clay, talc, diatomaceous earth, zeolite, montmorillonite, bentonite, acid clay, activated clay, attapulgite, calcite, vermiculite, perlite, pumice, and silica sand; synthetic organic substances such as urea; salts such as calcium carbonate, sodium carbonate, sodium sulphate, slaked lime, and baking soda; synthetic inorganic substances such as amorphous silica such as white carbon and titanium dioxide; plant carriers such as wood flour, corn stalk (cob), walnut shell (nut shell), fruit core, chaff, sawdust, bran, soy flour, powdered cellulose, starch, dextrin, and sugars; and various polymeric carriers such as crosslinked lignin, cation gel, gelatin gelated by heat or a polyvalent metal salt, water-soluble polymer gel such as agar, chlorinated polyethylene, chlorinated polypropylene, polyvinyl acetate, polyvinyl chloride, ethylene-vinyl acetate copolymer, and urea-aldehyde resin.

Examples of the liquid carrier include aliphatic solvents (paraffins), aromatic solvents (for example, xylene, alkylbenzene, alkylnaphthalene, and solvent naphtha), mixed solvents (kerosene), machine oils (refined high-boiling aliphatic hydrocarbons), alcohols (for example, methanol, ethanol, isopropanol, and cyclohexanol), polyhydric alcohols (for example, ethylene glycol, diethylene glycol, propylene glycol, hexylene glycol, polyethylene glycol, and polypropylene glycol), polyhydric alcohol derivatives (for example, propylene glycol ether), ketones (for example, acetone, acetophenone, cyclohexanone, methylcyclohexanone, and γ-butyrolactone), esters (fatty acid methyl ester (coconut oil fatty acid methyl ester), ethylhexyl lactate, propylene carbonate, dibasic acid methyl esters (succinic acid dimethyl ester, glutamic acid dimethyl ester, and adipic acid dimethyl ester), nitrogen-containing carriers (N-alkylpyrrolidones), oils and fats (for example, coconut oil, soybean oil, and rapeseed oil), amide solvents [dimethylformamide, (N,N-dimethyloctaneamide, N,N-dimethyldecaneamide, 5-(dimethylamino)-2-methyl-5-oxo-valeric acid methyl ester, N-acylmorpholine-based solvents (for example, CAS NO. 887947-29-7)], dimethyl sulfoxide, acetonitrile, and water.

Examples of the nonionic surfactants include sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkylphenyl ether, polyoxyethylene dialkyl phenyl ether, polyoxyethylene alkyl phenyl ether formalin condensate, polyoxyethylene/polyoxypropylene block polymer, alkyl polyoxyethylene/polyoxypropylene block polymer ether, polyoxyethylene alkylamine, polyoxyethylene fatty acid amide, polyoxyethylene fatty acid bisphenyl ether, polyoxyethylene benzylphenyl (or phenylphenyl) ether, polyoxyethylene styrylphenyl (or phenylphenyl) ether, polyoxyethylene ether and ester type silicone and fluorosurfactants, polyoxyethylene castor oil, polyoxyethylene hydrogenated castor oil, and alkyl glycosides. Examples of the anionic surfactants include sulphates such as alkyl sulphate, polyoxyethylene alkyl ether sulphate, polyoxyethylene alkylphenyl ether sulphate, polyoxyethylene benzyl (or styryl) phenyl (or phenylphenyl) ether sulphate, polyoxyethylene, polyoxypropylene block polymer sulphate; sulfonates such as paraffin (alkane) sulfonate, α-olefin sulfonate, dialkyl sulfosuccinate, alkylbenzene sulfonate, mono- or dialkyl naphthalene sulfonate, naphthalene sulfonate-formalin condensate, alkyl diphenyl ether disulfonate, lignin sulfonate, polyoxyethylene alkyl phenyl ether sulfonate, and polyoxyethylene alkyl ether sulfosuccinic acid half ester; fatty acid salts such as fatty acids, N-methyl-fatty acid sarcosinates, and resin acids; phosphates such as polyoxyethylene alkyl ether phosphate, polyoxyethylene mono- or dialkyl phenyl ether phosphate, polyoxyethylene benzyl (or styryl) phenyl (or phenylphenyl) ether phosphate, polyoxyethylene/polyoxypropylene block polymer, phosphatidylcholine phosphatidylethanolimine (lecithin), and alkyl phosphates. Examples of the cationic surfactants include ammonium salts such as alkyltrimethylammonium chloride, methylpolyoxyethylene alkylammonium chloride, alkyl N-methylpyridinium bromide, mono- or dialkylmethylated ammonium chloride, alkylpentamethylpropylenediamine dichloride; and benzalkonium salts such as alkyldimethylbenzalkonium chloride, and benzethonium chloride (octylphenoxyethoxyethyl dimethylbenzylammonium chloride). The surfactant may be a biosurfactant. Examples of the biosurfactant include rhamnolipids, surfactins, cellobiose lipids, sophorolipids, mannosyl alditol lipids, trehalose lipids, glucose lipids, oligosaccharide fatty acid esters, serrawettins, lychenysins, arthrofactins, spiculisporic acids, corynomycolic acids, agaritic acids, and emulsans.

Examples of the other formulation aid include inorganic salts used as pH adjusters such as sodium and potassium; fluorine-based and silicon-based defoamers; water-soluble salts such as common salt; water-soluble polymers used as thickeners such as xanthan gum, guar gum, carboxymethyl cellulose, polyvinylpyrrolidone, carboxyvinyl polymer, acrylic polymer, polyvinyl alcohol, water-soluble polymers such as starch derivatives and polysaccharides; alginic acid and salts thereof; metal stearates, sodium tripolyphosphate, sodium hexametaphosphate used as disintegrating dispersants; preservatives; colorants; antioxidants; UV absorbers; chemical damage reducers; and deterioration inhibitors.

Some formulations are used as they are and some are diluted with a diluent such as water to a predetermined concentration before use. The concentration of compound (I) when diluted before use is preferably in the range from 0.0001 to 1 wt.%. The same applies to the tautomers of compound (I).

These formulations are prepared so that compound (I) is contained as an active ingredient in an amount of 0.1 to 90 wt.%, and more preferably 0.2 to 50 wt.%. The amount of compound (I) used is from 0.005 to 50 kg, and more preferably from 0.03 to 30 kg per 1 ha of agricultural and horticultural land such as fields, rice fields, orchards, and greenhouses. The same applies to the tautomers of compound (I). Since the concentration and quantity used differ depending on the form of the agent, time of use, usage method, usage location, target crops and the like, they may be increased or decreased without being limited to the above range.

### (Other active ingredients)

The plant growth regulator in the present embodiment may be used in combination with other known active ingredients to enhance the performance as a plant growth regulator. Examples of the other known active ingredients include active ingredients contained in known plant growth regulators, fungicides, insecticides, acaricides, nematicides, and herbicides.

Examples of the active ingredients of known plant growth regulators include oxidized glutathione, L-glutamic acid, L-proline, aminoethoxyvinylglycine, chlormequat, chlorpropham, cyclanilide, dikegulac, daminogit, ethefone, flurprimidol, flumetraline, forchlorfenuron, gibberellin, hydrazide maleate salt, mepiquat chloride, methylcyclopropene, benzylaminopurine, paclobutrazol, prohexadione, thidiazuron, tributylphosphorotrithioate, trinexapac-ethyl, and uniconazole.

Examples of effective components suitable for fungicidal use include sterol biosynthesis inhibitor compounds, benzimidazole compounds, succinate dehydrogenase inhibitor compounds (SDHI compounds), strobilurin compounds, phenylamide compounds, dicarboximide compounds, anilinopyrimidine compounds, multi-point compounds, antibiotics, carbamate compounds, quinoline compounds, organophosphorus compounds, and carboxyamide compounds.

Examples of the sterol biosynthesis inhibitor compounds include azaconazole, bitertanol, bromuconazole, difenoconazole, cyproconazole, diniconazole, fenbuconazole, fluquinconazole, flutriafol, hexaconazole, imazalil, imibenconazole, metconazole, ipconazole, myclobutanil, pefurazoate, penconazole, prochloraz, propiconazole, prothioconazole, epoxiconazole, simeconazole, tebuconazole, tetraconazole, triadimefon, triadimenol, triflumizole, triticonazole, flusilazole, oxpoconazole, mefentrifluconazole, ipfentrifluconazole, 1-((1H-1,2,4-triazol-1-yl) methyl)-5-(4-chlorobenzyl)-2-(chloromethyl)-2-methylcyclopentan-1-ol, methyl-2- ((1H-1,2,4-triazol-1-yl)methyl)-3-(4-chlorobenzyl)-2-hydroxy-1-methylcyclopentane-1-carboxylate, fenpropimorph, fenpropidine, spiroxamine, tridemorph, bupirimate, fenarimol, pyrifenox, pyrisoxazole, nuarimol, etaconazole, piperaline, nafthifine, fenpyrazamine, fenhexamid, terbinafine, and triforine.

Examples of the benzimidazole compounds include carbendazim, benomyl, thiabendazole, thiophanate, thiophanate methyl, and fuberidazole.

Examples of the succinate dehydrogenase inhibitor compounds (SDHI compounds) include bixafen, benzovindiflupyr, boscalid, fluopyram, flutolanil, fluxapyroxad, furametpyr, isofetamide, isopyrazam, mepronil, penflufen, penthiopyrad, sedaxane, thifluzamide, fluindapyr, pyraziflumide, pydiflumetofen, pyraziflumide, benodanil, carboxin, pyrapropoyne, inpyrfluxam, isoflucypram, and oxycarboxin.

Examples of the strobilurin compounds include azoxystrobin, dimoxystrobin, enestroburin, fenamistrobin, fluoxastrobin, kresoxime methyl, metominostrobin, orysastrobin, picoxystrobin, pyraclostrobin, trifloxystrobin, mandestrobin, pyribencarb, pyraoxystrobin, pyrametostrobin, flufenoxystrobin, enoxastrobin, coumoxystrobin, triclopyricarb, fenaminstrobin, and metyltetraprole.

Examples of the phenylamide compounds include benalaxyl, benalaxyl M or chiralaxyl, metalaxyl, metalaxyl M or mefenoxam, and oxadixyl.

Examples of the dicarboximide compounds include procymidone, iprodione, and vinclozolin.

Examples of the anilinopyrimidine compounds include cyprodinil, mepanipyrim, and pyrimethanil.

Examples of the multi-point compounds include mancozeb, maneb, metiram, propineb, thiram (thiuram), zineb, ziram, amobam, anilazine, dithianon, fluazinam, pencycuron, quintozene, tolylfluanid, dodine, guazatine, iminoctadine (iminoctadine acetate and iminoctadine albesylate), copper, copper compounds [for example, basic copper chloride, cupric hydroxide, basic copper sulphate, copper sulphate, organic copper (oxine copper), copper nonylphenol sulfonate, and DBEDC], hydrogen carbonate (sodium hydrogen carbonate and potassium hydrogen carbonate), metallic silver, fentin, sulfur, mineral oil, baking soda, potassium carbonate, farbum, captan, captafol, fluoroimide, metasulfocarb, dipymetitrone, chlorothalonil (TPN), and folpet.

Examples of the antibiotics include kasugamycin, polyoxin, streptomycin, validamycin, and oxytetracycline.

Examples of the carbamate compounds include benthiavalicarb (benthiavalicarb-isopropyl), diethofencarb, iprovalicarb, propamocarb, and tolprocarb.

Examples of the quinoline compound include oxolinic acid, pyroquilon, quinoxyfen, and tebufloquin.

Examples of the organophosphorus compounds include dinocap, edifenphos (EDDP), fosetyl (fosetyl-aluminum), iprobenfos (IBP), meptyldinocap, and tolclofos-methyl.

Examples of the carboxyamide compounds include carpropamide, ethaboxam, fenoxanil, silthiofam, tiadinil, and isotianil.

Examples of other compounds for fungicidal use include ametoctradine, amisulbrom, cyazofamid, cyflufenamid, cymoxanil, diclocymet, diclomezine, famoxadone, fenamidone, fenitropan, fludioxonil, fluopicolide, flusulfamide, flutianil, harpin, isoprothiolane, isotianil, mandipropamid, metrafenone, oxathiapiprolin, phthalide, proquinazid, valifenalate, zoxamide, fenpicoxamid, picarbutrazox, quinofumelin, dimethomorph, flumorph, pyrimorph, ferimzone, acibenzolar (acibenzolar-S-methyl), etridiazole, hymexazole, probenazole, tricyclazole, tecloftalam, hydroxyisoxazole, fluoroimide, pyriofenone, diflumetorim, quinomethionate, aminopyrifene, dichlobentiazox, pyridachlometyl, ipflufenoquin, fluopimomide, florylpicoxamide, fluoxapiprolin, fenfuram, binapacryl, meptyldinocap, triphenyltin acetate, triphenyltin chloride, triphenyltin hydroxide, furalaxyl, ofurace, dimethirimol, ethirimol, octhilinone, chlozolinate, dimetachlone, fenpiclonil, blasticidin, triazoxide, dodine, picarbutrazox, pyrazophos, biphenyl, chloroneb, dicloran, tecnazene (TCNB), prothiocarb, natamycin, laminarin, flubeneteram, phosphoric acid, phosphate, shiitake mycelium extracts, and biopesticides (e.g., Agrobacterium radiobacter, Pseudomonas fluorescens, Pseudomonas rhodesia, Bacillus subtilis, Bacillus simplex, Bacillus amyloliquefaciens, non-pathogenic Erwinia carotovora, Lactobacillus plantarum, and Variovorax paradoxus).

Examples of effective components suitable for insecticidal use include organophosphorus compounds, carbamate compounds, pyrethroid compounds, nereistoxin compounds, neonicotinoid compounds, benzoylurea compounds, other insect growth control compounds, organic chlorine compounds, and compounds derived from natural products.

Examples of the organophosphorus compounds include acephate, azinphos-methyl, cadusafos, chlorethoxyfos, chlorfenvinphos, chlorpyrifos, cyanophos, demeton-S-methyl, diazinon, dichlorvos (DDVP), dicrotophos, dimethoate, disulfoton, ethione, ethoprophos, EPN, fenamiphos, fenitrothion (MEP), fenthion (MPP), fosthiazate, imicyafos, isofenphos, isoxathion, malathion, methamidophos, methidathion, mevinphos, monocrotophos, omethoate, oxydemeton-methyl, parathion, parathion-methyl, phenthoate, phorate, phosalone, phosmet, phosphamidone, phoxim, pirimiphos-methyl, profenofos, prothiophos, pyraclofos, pyridaphenthion, quinalphos, tebupirimfos, terbufos, triazophos, and trichlorfon (DEP).

Examples of the carbamate compounds include alanycarb, aldicarb, benfuracarb, BPMC, carbaryl (NAC), carbofuran, carbosulfan, cartap, fenoxycarb (BPMC), formetanate, isoprocarb (MIPC), methiocarb, methomyl, oxamyl, pirimicarb, thiodicarb, XMC, bendiocarb, ethiofencarb, fenobucarb, fenothiocarb, furathiocarb, metolcarb, and xylylcarb.

Examples of the pyrethroid compounds include acrinathrin, allethrin, cypermethrin, bifenthrin, cycloprothrin, cyfluthrin, cypermethrin, deltamethrin, dimefluthrin, esfenvalerate, etofenprox, fenpropathrine, fenvalerate, flubrocythrinate, flucythrinate, fluvalinate, halfenprox, cyhalothrin, metofluthrin, monfluorothrin, permethrin, profluthrin, tefluthrin, tralomethrin, cyfluthrin, kappa-bifenthrin, imiprothrin, pyrethrin, chloroprallethrin, epsilon-metofluthrin, epsilon-momfluorothrin, and cyphenothrin.

Examples of the nereistoxin compounds include cartap, bensultap, thiocyclam, monosultap, and bisultap.

Examples of the neonicotinoid compounds include acetamiprid, clothianidin, dinotefuran, imidacloprid, nitenpyram, thiacloprid, and thiamethoxam.

Examples of the benzoylurea compound include bistrifluron, chlorfluazuron, diflubenzuron, flucycloxuron, flufenoxuron, hexaflumuron, lufenuron, novaluron, noviflumuron, teflubenzuron, and triflumuron.

Examples of other insect growth control compounds include buprofezin, chromafenozide, cyromazine, halofenozide, methoxyfenozide, tebufenozide, and pyriproxyfen.

Examples of the organic chlorine compounds include aldrin, dieldrin, endosulfan, methoxychlor, lindane, and DDT.

Examples of the compounds derived from natural products include abamectin, live spores and produced crystal toxins derived from Bacillus thuringiensis, and mixtures thereof, bensultap, emamectin benzoate, lepimectin, milbemectin, spinetoram, spinosad, machine oil, starch, saccharified reduced starch, rapeseed oil, sodium oleate, propylene glycol monofatty acid ester, fatty acid glyceride, and ferric phosphate.

Examples of other compounds for insecticidal use include avermectin, chlorantraniliprole, tetrachlorantraniliprole, chlorfenapyr, cyantraniliprole, diafenthiuron, ethiprole, fipronil, flonicamid, flubendiamide, fluensulfone, flupyradifurone, indoxacarb, metaflumizone, metaldehyde, pymetrozine, pyridalyl, pyrfluquinazon, silafluofen, spirotetramat, sulfoxaflor, tolfenpyrad, afidopyropen, broflanilide, cyclaniliprole, dicloromezotiaz, flometoquin, fluazaindolizine, fluhexafon, fluxametamide, pyriprole, tetraniliprole, triflumezopyrim, methoprene, tyclopyrazoflor, flupyrimin, spiropidion, benzpyrimoxan, cyhalodiamide, sulfluramid, isocycloseram, DNOC, rotenone, nicofluprole, and dimpropyridaz.

Examples of active ingredients suitable for acaricidal use (acaricidal active ingredient) include acequinocyl, amidoflumet, amitraz, azocyclotin, bifenazate, bromopropylate, chlorfenson, chinomethionate, phenisobromolate, benzoximate, clofentezine, cyenopyrafen, cyflumetofen, cyhexatin, diflubenzuron, dienochlor, etoxazole, fenazaquin, fenbutatin oxide, fenpyroximate, fenothiocarb, fluacrypyrim, hexythiazox, propargite (BPPS), pyflubumide, pyridaben, pyrimidifen, spirodiclofen, spiromesifen, tebufenpyrad, tetradifon, acynonapyr, cyetpyrafen, flupentiofenox, and blended oils.

Examples of the most suitable active ingredient for nematicidal use (nematicidal active ingredient) include D-D (1,3-dichloropropene), DCIP (dichlorodiisopropyl ether), methyl isothiocyanate, carbam sodium salt, cadusafos, fosthiazate, imicyafos, morantel tartrate, levamisole hydrochloride, nemadectin, cyclobutrifluram, and tioxazafen.

Examples of effective components suitable for herbicidal use include acetolactate synthesis (ALS) inhibitor compounds, amino acid compounds, cyclohexanedione compounds, acetamide compounds, bipyridinium compounds, allyloxyphenoxypropionic acid compounds, carbamates compounds, pyridine compounds, urea compounds, dinitroaniline compounds, protoporphyrinogen oxidase (PPO) inhibitor compounds, phenoxyacetic acid compounds, hydroxyphenylpyruvate dioxygenase enzyme (HPPD) inhibitor compounds, and triazine compounds.

Examples of the acetolactate synthesis (ALS) inhibitor compounds include imazamethabenz and imazamethabenz-methyl, imazamox, imazapic, imazapyr, imazaquin, imazethapyr, amidosulfuron, azimsulfuron, bensulfuron and bensulfuron methyl, chlorimuron and chlorimuron methyl, chlorsulfuron, cinosulfuron, cyclosulfamurone, ethametsulfuron and ethametsulfuron-methyl, ethoxysulfuron, flazasulfuron, flucetosulfuron, flupyrsulfuron, foramsulfuron, halosulfuron and halosulfuron-methyl, imazosulfuron, iodosulfuron and iodosulfuron-methyl, mesosulfuron, metazosulfuron, metsulfuron and metsulfuron-methyl, nicosulfuron, oxasulfuron, primisulfuron and primisulfuron-methyl, propyrisulfuron, prosulfuron, pyrazosulfuron and pyrazosulfuron ethyl, rimsulfuron, sulfometuron and sulfometuron methyl, sulfosulfuron, thifensulfuron and thifensulfuron methyl, triasulfuron, tribenuron, trifloxysulfuron, triflusulfuron and triflusulfuron methyl, tritosulfuron, bispyribac sodium, cloransulam and cloransulam-methyl, diclosulam, florasulam, flucarbazone and salts thereof, flumetsulam, metosulam, orthosulfamurone, penoxsulam, propoxycarbazone and salts thereof, pyribenzoxime, pyriftalid, pyriminobac-methyl, pyrimisulfan, pyrithiobac and salts thereof, pyroxsulam, thiencarbazone and thiencarbazone-methyl, and triafamon.

Examples of the amino acid compounds include bialaphos and salts thereof, glufosinate and salts thereof, glufosinate P and salts thereof, and glyphosate and salts thereof.

Examples of the cyclohexanedione compounds include butroxydim, clethodim, cycloxydim, profoxydim, sethoxydim, tepraloxydim, and tralkoxidym.

Examples of the acetamide compounds include napropamide, dimethachlor, pethoxamid, acetochlor, alachlor, butachlor, dimethenamid and dimethenamid P, metazachlor, metolachlor and S-metolachlor, pretilachlor, propachlor, thenylchlor, flufenacet, and mefenacet.

### Examples of the bipyridinium compounds include diquat and paraquat.

Examples of the allyloxyphenoxypropionic acid compounds include clodinafop and clodinafop propargyl, cyhalofop butyl, diclofop and diclofop-methyl and diclofop-p-methyl, fenoprop and fenoprop-ethyl and fenoprop-p-ethyl, fluazifop and fluazifop-butyl and fluazifop-p-butyl, haloxyfop and haloxyfop methyl and haloxyfop-p-methyl, metamifop, propaquizafop and quizalofop and quizalofop-ethyl and quizalofop-p-ethyl, and quizalofop-P-tefuryl.

Examples of the carbamate compounds include asulam, carbetamide, desmedipham, phenmedipham, butyrate, EPTC, esprocarb, molinate, orbencarb, prosulfocarb, pyributicarb, thiobencarb (benchiocarb) and tri-allate.

Examples of the pyridine compounds include aminopyralid, clopyralid, diflufenican, dithiopyr, fluridone, fluroxypyr, halauxifen, picloram and salts thereof, picolinafen, thiazopyr, triclopyr, and salts thereof.

Examples of the urea compounds include chlorotoluron, dimuron, diuron (DCMU), fluometuron, isoproturon, linuron, methabenzthiazuron, tebuthiuron, cumyluron, karbutilate, and isouron.

Examples of the dinitroaniline compounds include benfluralin (bethrodine), butralin, ethalfluralin, oryzalin, pendimethalin, prodiamine, and trifluralin.

Examples of the protoporphyrinogen oxidase (PPO) inhibitor compounds include acifluorfen, aclonifene, azafenidin, bifenox, chlomethoxynil, ethoxyfen and ethoxyfen-ethyl, fomesafen, fluazolate, fluoroglycofen and fluoroglycofen-ethyl, halosafen, lactofen, oxyfluorfen, butafenacil, carfentrazone and carfentrazone-ethyl, cinidon-ethyl, flumiclorac-pentyl, flumioxazin, fluthiacet and fluthiacet-methyl, oxadiargyl, oxadiazon, pentoxazone, pyraclonil, pyraflufen and pyraflufen-ethyl, saflufenacil, sulfentrazone, thidiazimine, benzfendizone, profluazole, and flufenpyr-ethyl.

Examples of the phenoxyacetic acid compound include 2,4-D and salt thereof, 2,4-DB and salt thereof, clomeprop, dichlorprop, MCPA and salt thereof, MCPB and salt thereof, and mecoprop (MCPP) and salt thereof, and mecoprop P and salt thereof.

Examples of the hydroxyphenylpyruvate dioxygenase enzyme (HPPD) inhibitor compounds include benzobicyclon, benzofenap, bicyclopyrone, isoxaflutol, mesotrione, pyrasulfotol, pyrazolynate (pyrazolate), pyrazoxyfen, sulcotrione, tefuryltrione, tembotrione, topramezone, fenquinotrione, and tolpyralate.

Examples of the triazine compound include ametryn, atrazine, cyanazine, dimethametryn, hexazinone, indaziflam, metamitron, metribuzin, prometryn, simazine (CAT), simetryn, terbuthylazine, terbutryn, and triaziflam.

Examples of other compounds for herbicidal use include amicarbazone, aminocyclopyrachlor, aminotriazole, anilofos, beflubutamid, benazolin, benfuresate, bentazone, bromacil, bromobutide, bromoxynil, butamifos, cafenstrole, chloridazon (PAC), chlorthal, clomazone, cumyluron, dicamba (MDBA) and its salts, dichlobenil (DBN), difenzoquat, diflufenzopyr, endothal and its salts, ethofumesate, etobenzanid, fenoxasulfone, fentrazamide, flupoxam, fluorochloridone, flurtamone, indanofan, ioxynil, ipfencarbazone, isoxaben, lenacil, methylarsonic acid, naptalam, norflurazon, oxaziclomefone, pinoxaden, propanil, propyzamide, pyridate, pyroxasulfone, promacyl, quinclorac, quinmerac, quinoclamin, terbacil, cyclopyrimorate, florpyrauxifen-benzyl, lancotrione, epyrifenacil, dimesulfazet, tetflupyrolimet and its salts, tiafenacil, trifludimoxazin, tetrapion (flupropanate) and its salts, and d-limonene.

### Method for promoting plant growth

The plant growth regulator in the present embodiment may be used, for example, in cultivated lands such as fields, paddy fields, lawns, and orchards or non-cultivated lands. The plant growth regulators in the present embodiment can be used by all methods of fertilization, such as spraying on stems and leaves, mixing into water supply, spraying on soil, injecting into subsoil using an injector, seed treatment including treatment of bulbs and tubers, and direct fertilization to plants. Therefore, the method for promoting plant growth in the present embodiment includes a procedure for fertilizing using the above-mentioned plant growth regulator.

In the case of application by mixing with feed water, for example, water is fed to the crop, or the water surface of a paddy field may be treated with granules or the like. In one example, the concentration of the active ingredient in the feed water is from 0.5 to 500 mg/L, and preferably from 1 to 300 mg/L. The amount of the active ingredient used when administered to paddy water is, for example, from 0.5 to 5000 g, and preferably from 3 to 3000 g per 10a of paddy field.

In the case of application by foliar application or application to soil, a planting hole or the vicinity thereof may be treated with granules or the like in the transplantation of seedling or the like, or seeds or the earth around a plant may be treated with granules, a wettable powder, or the like. In addition, it may be preferable to mix with soil after spraying on the soil. The amount of the active ingredient used for foliar application or application to the soil surface is, for example, from 0.5 to 5000 mg, and preferably from 3 to 3000 mg per 1 m² of agricultural and horticultural land.

In seed treatment, the agent is applied to the seeds by mixing and stirring wettable powders and dustable powders with the seeds or by dipping the seeds in diluted wettable powders. The seed treatment also includes seed coating treatments. The amount of active ingredients used in the case of seed treatment is, for example, from 0.005 to 10,000 g, and preferably from 0.05 to 1,000 g per 100 kg of the seeds. Seeds treated with agricultural or horticultural chemicals can be used in the same way as common seeds.

Additionally, since the concentration and quantity used differ depending on the form of the agent, time of use, usage method, usage location, target crops and the like, they may be increased or decreased within the above ranges. As described above, compound (I) and its tautomer exhibit an excellent growth promoting effect on a wide range of plants.

### [Use of plant growth regulator]

The plant growth regulators in this embodiment exhibit an excellent growth promoting effect in treated plants, as described above. Therefore, the plant growth regulator in this embodiment can be used, for example, as biostimulants and fertilizers. The plant growth regulator in this embodiment may also be mixed with soil conditioners and pesticides for use.

The term "fertilizer" is primarily intended for anything that acts on plants or soil for the purpose of supplying nutrients to plants or causing chemical changes in the soil. The term "biostimulant" is primarily intended for anything that acts on plant physiology through a different pathway than nutrients for the purpose of improving crop vitality, yield, and quality.

### [Summary]

As described above, the plant growth regulator according to the present invention includes a compound represented by Formula (I) or its tautomer, or an agrochemically acceptable salt thereof as an active ingredient: 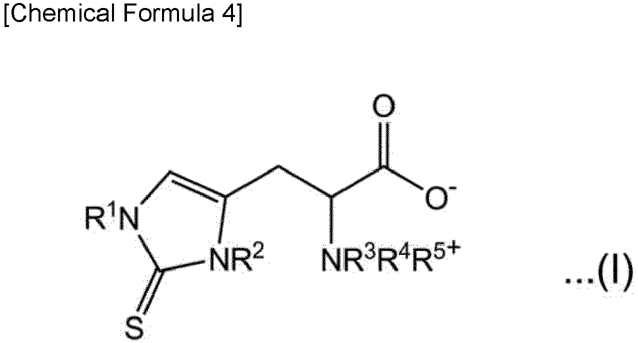 where in Formula (I), R¹ and R² each independently represent a hydrogen atom or an alkyl group having from 1 to 4 carbon atoms, and R³ to R⁵ each independently represent an alkyl group having from 1 to 4 carbon atoms.

The plant growth regulator according to the present embodiment preferably includes a compound represented by Formula (I) or an agrochemically acceptable salt thereof as an active ingredient.

In the plant growth regulator according to the present invention, in Formula (I), at least one of R¹ and R² is preferably a hydrogen atom.

Additionally, in the plant growth regulator according to the present invention, in Formula (I), it is preferred that R¹ and R² are hydrogen atoms, and R³ to R⁵ are methyl groups.

In the plant growth regulator according to the present invention, the compound represented by Formula (I) is preferably L-(+)-ergothioneine.

The method for promoting plant growth according to the present invention includes treating a plant with the compound represented by Formula (I) or its tautomer, or an agrochemically acceptable salt thereof.

Embodiments of the present invention will be described in further detail hereinafter using examples. The present invention is not limited to the examples below, and it goes without saying that various aspects are possible with regard to the details thereof. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents described in the present specification are herein incorporated by reference.

### [EXAMPLES]

### Example 1

### Sample

*Arabidopsis thaliana* (Col-O) was sown in plastic pots having a width of 65 mm, a depth of 65 mm, and a height of 70 mm, three individuals per pot. Plastic deep dishes each having a diameter of 160 mm and a height of 28 mm were prepared, and three pots were respectively placed therein. As soil, 100 mL of vermiculite, 50 mL of granular soil (JA Granular Kumiai Synthetic Soil No. 3), and 50 mL of vermiculite were placed in each pot in this order.

### Control conditions

In a thermostatic chamber set at room temperature of 25°C, the light period was 16 hours and the dark period was 8 hours. The light conditions were set using a fluorescent lamp (PLANT FLEC, 40 W LED fluorescent lamp for plant growth, electric bulb color, available from Nippon Medical and Chemical Instruments Co., Ltd.) so that the light intensity was 5000 Ix in the central part under the fluorescent lamp irradiation. Water supply was done from the bottom, and the water level was set at about 5 mm. Ergothioneine treatment was started 4 weeks after seeding. More specifically, on the 21st, 23rd, 25th, and 27th days after seeding, 50 mL of a 1 mM L-(+)-ergothioneine (available from Cayman Chemical) aqueous solution was added instead of water supply.

### Verification

On the 37th day after seeding, the plant height (cm) and the number of flowers and fruits per *Arabidopsis thalianawere* measured. The results are shown in Table 1.

### Comparative Example 1

The same operation as in Example 1 was carried out except that L-(+)-ergothioneine in the aqueous solution was replaced with oxidized glutathione (available from Wako Pure Chemical Industries, Ltd.).

### Comparative Example 2

The same operation as in Example 1 was carried out except that the L-(+)-ergothioneine aqueous solution was replaced with distilled water.

### Example 2

### Sample

One individual *Arabidopsis thaliana* (Col-O) was sown in a plastic pot having a diameter of 60 mm and a height of 55 mm. Plastic deep dishes each having a diameter of 160 mm and a height of 28 mm were prepared, and six pots were respectively placed therein. As soil, 45 mL of vermiculite, 22.5 mL of granular soil (JA Granular Kumiai Synthetic Soil No. 3), and 22.5 mL of vermiculite were placed in each pot in this order.

### Control conditions

The procedure was the same as in Example 1, except that the room temperature was set to 22°C.

### Verification

On the 85th day after seeding, seeds were harvested, and the seed yield (mg/plant) was measured. The results are shown in Table 2.

### Example 3

The same operation as in Example 2 was carried out except that the concentration of L-(+)-ergothioneine aqueous solution was set to 0.1 mM.

### Example 4

The same operation as in Example 2 was carried out except that the concentration of L-(+)-ergothioneine aqueous solution was set to 0.01 mM.

### Comparative Example 3

The same operation as in Example 2 was carried out except that L-(+)-ergothioneine in the aqueous solution was replaced with oxidized glutathione (available from Wako Pure Chemical Industries, Ltd.).

### Comparative Example 4

The same operation as in Example 2 was carried out except that L-(+)-ergothioneine in the aqueous solution was replaced with L-glutamic acid (available from Wako Pure Chemical Industries, Ltd.).

### Comparative Example 5

The same operation as in Example 2 was carried out except that L-(+)-ergothioneine in the aqueous solution was replaced with L-proline (available from Wako Pure Chemical Industries, Ltd.).

### Comparative Example 6

The same operation as in Example 2 was carried out except that the L-(+)-ergothioneine aqueous solution was replaced with distilled water.

### Example 5

### Sample

The same operation as in Example 2 was carried out.

### Control conditions

The same operation as in Example 1 was carried out except that the room temperature was set at 22°C and L- (+)-ergothioneine was added 2 weeks after seeding, more specifically on the 8th, 10th, 12th, and 14th days.

### Verification

On the 82nd day after seeding, seeds were harvested, and the seed yield (mg/plant) was measured. The results are shown in Table 3.

### Example 6

The same operation as in Example 5 was carried out except that L- (+)-ergothioneine was added 4 weeks after seeding, more specifically on the 22nd, 24th, 26th, and 28th days.

### Comparative Example 7

The same operation as in Example 5 was carried out except that oxidized glutathione was used in place of L- (+)-ergothioneine in the aqueous solution and added 4 weeks after seeding, more specifically on the 22nd, 24th, 26th, and 28th days.

### Comparative Example 8

The same operation as in Example 5 was carried out except that the L-(+)-ergothioneine aqueous solution was replaced with distilled water.

### Analysis

The ratio of "grass height" and "flowers and fruits" for Example 1, Comparative Example 1, and Comparative Example 2 to the results of Comparative Example 2 are shown in Table 1 as "Ratio", respectively. The "seed yield" for Examples 2 to 4 and Comparative Examples 3 to 6 are shown as "Ratio" in Table 2, where the value for Comparative Example 6 is set to 1. The "seed yield" for Examples 5 and 6 and Comparative Examples 7 and 8 are shown as "Ratio" in Table 3, where the value for Comparative Example 8 is set to 1.

### [Table 1]

**Table 1**

| | Test compound | Plant height | | Flower and fruit | |
|---|---|---|---|---|---|
| | | (cm) | Ratio | (Number/plant) | Ratio |
| Example 1 | L-(+)-ergothioneine | 29 | 1.3 | 26 | 1.7 |
| Com parative Example 1 | Oxidized glutathione | 24 | 1.1 | 22 | 1.5 |
| Com parative Example 2 | - | 22 | 1.0 | 15 | 1.0 |

### [Table 2]

**Table 2**

| | Test compound | Compound concentration | Seed yield | |
|---|---|---|---|---|
| | | (mM) | (mg/plant) | Ratio |
| Example 2 | L-(+)-ergothioneine | 1 | 142 | 1.7 |
| Example 3 | L-(+)-ergothioneine | 0.1 | 130 | 1.5 |
| Example 4 | L-(+)-ergothioneine | 0.01 | 109 | 1.3 |
| Com parative Example 3 | Oxidized glutathione | 1 | 100 | 1.2 |
| Com parative Example 4 | L-glutamic acid | 1 | 90 | 1.0 |
| Com parative Example 5 | L-proline | 1 | 102 | 1.2 |
| Com parative Example 6 | - | - | 86 | 1.0 |

### [Table 3]

**Table 3**

| | Test compound | Time of treatment | Seed yield | |
|---|---|---|---|---|
| | | (Week) | (mg/plant) | Ratio |
| Example 5 | L-(+)-ergothioneine | 2 | 140 | 2.0 |
| Example 6 | L-(+)-ergothioneine | 4 | 114 | 1.7 |
| Com parative Example 7 | Oxidized glutathione | 4 | 92 | 1.3 |
| Com parative Example 8 | - | - | 69 | 1.0 |

## Claims

1. A plant growth regulator comprising a compound represented by Formula (I) or its tautomer, or an agrochemically acceptable salt thereof as an active ingredient: 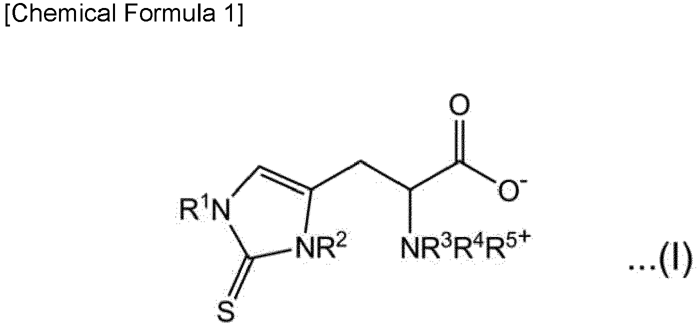 where in Formula (I), R¹ and R² each independently represent a hydrogen atom or an alkyl group having from 1 to 4 carbon atoms, and R³ to R⁵ each independently represent an alkyl group having from 1 to 4 carbon atoms.

2. The plant growth regulator according to claim 1, comprising a compound represented by Formula (I) or an agrochemically acceptable salt thereof as an active ingredient.

3. The plant growth regulator according to claim 1 or 2, wherein in Formula (I), at least one of R¹ and R² is a hydrogen atom.

4. The plant growth regulator according to any one of claims 1 to 3, wherein in Formula (I), R¹ and R² are hydrogen atoms, and R³ to R⁵ are methyl groups.

5. The plant growth regulator according to any one of claims 1 to 4, wherein the compound represented by Formula (I) is L-(+)-ergothioneine.

6. A method for promoting plant growth comprising treating a plant with a compound represented by Formula (I) or its tautomer, or an agrochemically acceptable salt thereof: 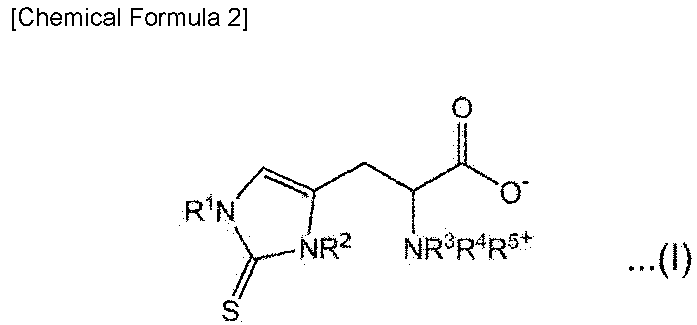 where in Formula (I), R¹ and R² each independently represent a hydrogen atom or an alkyl group having from 1 to 4 carbon atoms, and R³ to R⁵ each independently represent an alkyl group having from 1 to 4 carbon atoms.
